# EUROPÄISCHE PATENTSCHRIFT

(11) **EP 1 056 721 B1**
(45) Veröffentlichungstag und Bekanntmachung des Hinweises auf die Patenterteilung: **14.07.2004**
(21) Anmeldenummer: 99908885.9
(22) Anmeldetag: 12.02.1999
(51) Int. Cl.: C07D 209/52

(54) **VERFAHREN ZUR AUFREINIGUNG VON (+-)2-AZABICYCLO (2.2.1)HEPT-5-EN-3-ON**
METHOD FOR PURIFYING (+-)-2-AZABICYCLO(2.2.1)HEPT-5-EN-3-ONE
PROCEDE D'EPURATION DE (+-)-2-AZABICYCLO(2.2.1)HEPT-5-EN-3-ONE

(30) Priorität: 27.02.1998 DE 19808136
(43) Veröffentlichungstag der Anmeldung: 06.12.2000
(73) Patentinhaber: Degussa AG, 40474 Düsseldorf (DE)
(72) Erfinder: DE SCHRIJVER, Johny, B-9111 Nieuwkerken (BE); OTTEN, Philippe, B-2547 Lint (BE)
(86) Internationale Anmeldenummer: PCT/EP1999/000933
(87) Internationale Veröffentlichungsnummer: WO 1999/043655

(56) Entgegenhaltungen:
- EP-A- 0 508 352
- EP-A- 0 533 048
- DE-A- 19 625 323

## Beschreibung

Die vorliegende Erfindung betrifft ein verbessertes Verfahren zur Aufreinigung von (±)-2-Azabicyclo[2.2.1]hept-5-en-3-on aus seiner Reaktionslösung.

(±)-2-Azabicyclo[2.2.1]hept-5-en-3-on ist Ausgangsmaterial zur Herstellung von karbozyklischen Nukleosidanalogen, die wegen ihrer antiviralen und chemotherapeutischen Eigenschaften als Heilmittel in der Medizin von Interesse sind.

Die Herstellung von (±)-2-Azabicyclo[2.2.1]hept-5-en-3-on wird beispielsweise in der EP 0 508 352 erwähnt. Das dort genannte Verfahren benutzt zur Herstellung der gewünschten Verbindung allerdings ausschließlich organische Lösungsmittel. Vorteilhaft an diesem Verfahren ist die hohe Reinheit, mit der das (±)-2-Azabicyclo[2.2.1]hept-5-en-3-on gewonnen werden kann. Nachteilig ist allerdings die Verwendung von organischen Lösungsmitteln bei dieser Reaktion. So arbeiten vorteilhafte moderne Verfahren alle in Wasser als Reaktionslösungsmittel.

Der Vorteil dieser modernen Variante ist in der Möglichkeit zu sehen, die gesamte Synthese ausgehend von einem geeigneten Sulfinat, Cyclopentadien und Chlorcyan als Eintopfverfahren durchführen zu können. Da ein Produkt der Reaktion wiederum das eingesetzte Sulfinat ist, kann dieses erfolgreich im Kreis gefahren werden, wodurch die Einsatzkosten des Verfahrens wesentlich gesenkt werden können (EP 0 533 048 und DE 196 25 323).

In den beiden eben genannten Fällen erfolgt die Aufreinigung der derart vorteilhaft hergestellten Reaktionslösung durch Extraktion der Produkte in ein organisches Lösungsmittel bei pH-Werten der wäßrigen Phase von ca. 8.

Die organische Produktphase wird anschließend laut EP 0 533 048 zur Isolation des Produktes eingeengt und getrocknet. Das erhaltene Material ist orange bzw. gelbbraun gefärbt und hat einen Gehalt von <96 % (HPLC).

Laut DE 196 25 323 kann (±)-2-Azabicyclo[2.2.1]hept-5-en-3-on aus der organischen Phase nach Aufkonzentrieren kristallisiert werden. Die Kristallisation bedingt je nach Lage des Löslichkeitsgleichgewichts mehr oder minder große Ausbeuteverluste. So ergeben sich nach dieser Aufreinigungsstrategie Ausbeuten an hellbräunlich gefärbtem Produkt von ≤ 75 % mit Gehalten von 98-99 % (HPLC).

Das betrachtete (±)-2-Azabicyclo[2.2.1]hept-5-en-3-on ist, wie oben beschrieben, ein Zwischenprodukt zur Herstellung von Arzneimitteln. Im Hinblick auf die Reinheitsanforderungen, die an ein zugelassenes Medikament. gestellt werden, ist mithin ein Zwischenprodukt zur Synthese des Wirkstoffs zu bevorzugen, das qualitativ besonders vorteilhaft ist. Qualitativ vorteilhaft ist ein möglichst farbloses Zwischenprodukt mit einem möglichst geringen Nebenproduktanteil.

Gleichzeitig soll das Zwischenprodukt jedoch preisgünstig herzustellen sein. D. h., es darf nicht durch komplizierte und teure Aufreinigungsprozedere gereinigt werden müssen, und die Ausbeute an verkaufsfähigem Material soll möglichst hoch sein.

Aufgabe der vorliegenden Erfindung ist es deshalb, ausgehend von einer Herstellung von (±)-2-Azabicyclo[2.2.1]hept-5-en-3-on in wäßriger Lösung, eine Aufreinigungsstrategie zu entwickeln, welche es gestattet, ein Produkt zu generieren, das weitestgehend von Nebenprodukten befreit und möglichst farblos ist.

Eine weitere Aufgabe besteht darin, das Produkt durch diese Aufreinigungsstrategie bei verbesserter Produktqualität in mit dem Stand der Technik vergleichbaren Ausbeuten bereitzustellen.

Eine weitere Aufgabe ist darin zu sehen, die Aufreinigungsprozedur so zu gestalten, daß sie möglichst unkompliziert und damit ökonomisch vorteilhaft durchgeführt werden kann.

Diese und weitere nicht näher genannte, sich jedoch aus dem Stand der Technik unmittelbar ergebende Aufgaben werden durch ein Aufreinigungsverfahren, welches Gegenstand des vorliegenden Anspruchs 1 ist, gelöst. Vorteilhafte Varianten werden in den von Anspruch 1 abhängigen Unteransprüchen unter Schutz gestellt.

Dadurch, daß eine wäßrige aus einem wäßrigen Herstellprozeß stammende Reaktionslösung von (±)-2-Azabicyclo[2.2.1]hept-5-en-3-on mit einem organischen Lösungsmittel extrahiert wird, die Phasen getrennt werden und man die produkthaltige organische Lösungsmittelphase anschließend mit Wasser reextrahiert, gelangt man zu einer Lösung von (±)-2-Azabicyclo[2.2.1]hept-5-en-3-on in Wasser, welche 97 % der theoretisch möglichen Menge an Produkt erhält. Nach Abtrennung des Wassers und Trocknung des so aufgereinigten Materials ergibt sich ein weißes Pulver, welches eine Reinheit von ≥ 99 % (HPLC) aufweist.

Völlig überraschend und dennoch vorteilhaft gelangt man mit dem erfindungsgemäßen Aufreinigungsprozeß zu einem qualitativ überragenden Material, welches so aus dem Stand der Technik bei diesem Herstellverfahren nicht bekannt war. Die Ausbeuteverluste bei dieser Aufreinigungsstrategie sind so gering, daß trotz der immens verbesserten Produktqualität die Ausbeute vergleichbar mit denen des Standes der Technik ist.

(±)-2-Azabicyclo[2.2.1]hept-5-en-3-on löst sich sehr gut in Wasser. Durch die oben beschriebene Aufreinigung erhält man eine wäßrige Lösung des erfindungsgemäß gereinigten Produkts, welche nach Belieben aufkonzentriert werden kann und so als konzentrierte wäßrige Lösung verkauft werden kann.

Diese Maßnahme ist sehr vorteilhaft, da eine technisch komplizierte und ökonomisch nachteilige Kristallisation und Trocknung des Produktes entfallen kann. Dieser äußerst überraschende und dennoch sehr vorteilhafte Effekt ist durch die gute Abtrennung der Nebenprodukte der Reaktion durch den einfachen der organischen Extraktion nachgeschalteten Phasenwechsel des Produkts bedingt. Dies war keineswegs voraussehbar, da mithin üblicherweise eine Kristallisation als die im Hinblick auf Kosten und Reinigungseffekt optimale Reinigungsmethode für Feststoffe angesehen wird.

Bevorzugt kann man die Extraktionsschritte mehrmals hintereinander ausführen, wodurch die Ausbeuteverluste minimiert werden. Gegebenenfalls kann bis zu achtmal hintereinander extrahiert werden. Ebenso vorteilhaft ist eine kontinuierliche Extraktion. Zur weiteren Ausbeuteverminderung ist es vorteilhaft, die organische produkthaltige Phase vor der zweiten Extraktion mit Wasser ggf. unter vermindertem Druck einzuengen. Ganz besonders bevorzugt ist es, die aus dem Herstellprozeß stammende wäßrige Produktphase vor der Extraktion mit dem organischen Lösungsmittel mit Natriumchlorid zu versetzen. Die Zugabe des Natriumchlorids kann ggf. bis zur Sättigungsgrenze hin erfolgen. Dadurch werden die organischen Produkte der Reaktion möglichst vollständig bei der Extraktion in der organischen Phase gelöst.

Falls gewünscht, kann das (±)-2-Azabicyclo[2.2.1]hept-5-en-3-on aus der wäßrigen finalen Produktphase durch Eindampfen und Trocknen isoliert werden.

Als organische Lösungsmittel können für die primäre Extraktion alle organischen Lösungsmittel verwendet werden, welche sich mit Wasser nicht mischen, wie chlorierte Kohlenwasserstoffe, Ketone, Ether und dergleichen. Bevorzugt sind solche, die darüber hinaus einen niedrigen Siedepunkt aufweisen, da andernfalls ein Eindampfen der organischen Phase erschwert wird. Als bevorzugte Lösungsmittel kommen in Betracht Methylenchlorid, Methyltert.butylether, Chloroform, Methylisobutylketon, Methylisopropylketon, Nitromethan, Nitroethan, 1- und 2-Nitropropan.

Besonders bevorzugt ist der Einsatz von MTBE.

Die Möglichkeit der Herstellung einer sehr reinen wäßrigen Lösung von (±)-2-Azabicyclo[2.2.1]hept-5-en-3-on ist ursächlich dafür, das Produkt in ökonomisch wie ökologisch gegenüber dem Stand der Technik vorteilhafter Weise generieren zu können, da es zusätzliche kostspielige Reinigungsschritte vermeidet damit Kosten und Energie sparen hilft.

Die folgenden Beispiele sollen die Erfindung verdeutlichen, sie jedoch keinesfalls einengen.

### Beispiel 1

10 kg dunkelbraune wässrige Reaktionslösung (enthält 1,2 kg (±)-2-Azabicyclo[2.2.1]hept-5-en-3-on) wird mittels 30,7 kg MTBE (8 Extraktionsstufen) extrahiert. Die MTBE-Lösung wird bis auf eine 50%ige Lösung (1,2 kg (±)-2-Azabicyclo[2.2.1]hept-5-en-3-on in 1,2 kg MTBE) eingedampft. 2,4 kg Wasser wird zugefügt. Die Phasen werden heftig gerührt und nachher getrennt. Die organische Phase enthält 2,5 % vom eingesetzten (±)-2-Azabicyclo[2.2.1]hept-5-en-3-on. Die wässrige Phase wird bis auf eine leichtgelbe 60%ige (±)-2-Azabicyclo[2.2.1]hept-5-en-3-on-Lösung eingedampft.

Nach Wasserabtrennung und Trocknung bei verringertem Druck erhält man 1,17 kg gebrochen weißes (±)-2-Azabicyclo[2.2.1]hept-5-en-3-on (Reinheit 99,3 % (HPLC)), entsprechend 97 % Ausbeute.

### Beispiel 2

580 g dunkelbraune wässrige Reaktionslösung (enthält 58 g 2-Azabicyclo-(2.2.1)-hept-5-en-3-on) wird mittels 3480 g Toluol (8 Extraktionsstufen) extrahiert. Die Toluollösung wird bis auf eine 37,2 %ige Lösung (30,5 g 2-Azabicyclo-(2.2.1)-hept-5-en-3-on in 51,5 g Toluol) eingedampft. 160 g Wasser werden zugefügt. Die Phasen werden heftig gerührt und nachher getrennt. Die organische Phase enthält 2,6 % von eingesetztem 2-Azabicyclo-(2.2.1)-hept-5-en-3-on. Die wässrige Phase wird bis auf eine leichtgelbe 60 %ige 2-Azabicyclo-(2.2.1)-hept-5-en-3-on-Lösung eingedampft. Nach Wasserabtrennung und Trocknung bei verringertem Druck erhält man 30,6 g gebrochen weißes 2-Azabicyclo-(2.2.1)-hept-5-en-3-on (Reinheit 99,6 % (HPLC)) entsprechend 53 % Ausbeute.

### Beispiel 3

573 g dunkelbraune wässrige Reaktionslösung (enthält 57,3 g 2-Azabicyclo-(2.2.1)-hept-5-en-3-on) wird mittels 6376 g Tetrachlormethan (8 Extraktionsstufen) extrahiert. Die Tetrachlormethanlösung wird bis auf eine 36,6 %ige Lösung (20,6 g 2-Azabicyclo-(2.2.1)-hept-3-en-3-on in 35,7 g Tetrachlormethan) eingedampft. 40 g Wasser werden zugefügt. Die Phasen werden heftig gerührt und nachher getrennt. Die organische Phase enthält 2,5 % von eingesetztem 2-Azabicyclo-(2.2.1)-hept-5-en-3-on. Die wässrige Phase wird bis auf eine leichtgelbe 60 %ige 2-Azabicyclo-(2.2.1)-hept-5-en-3-on-Lösung eingedampft. Nach Wasserabtrennung und Trocknung bei verringertem Druck erhält man 19,1 g gebrochen weißes 2-Azabicyclo-(2.2.1)-hept-5-en-3-on (Reinheit 99,1 % (HPLC)) entsprechend 33 % Ausbeute.

### Vergleichsbeispiel

10 kg dunkelbraune wässrige Reaktionslösung (enthält 1,2 kg (±)-2-Azabicyclo[2.2.1]hept-5-en-3-on) wird mittels 30,7 kg MTBE (8 Extraktionsstufen) extrahiert. Die MTBE-Lösung wird bis auf eine 25%ige Lösung (1,2 kg (±)-2-Azabicyclo[2.2.1]hept-5-en-3-on in 3,6 kg MTBE) eingedampft. Durch Abkühlungskristallisation und Trocknung bei verringertem Druck wird 0,9 kg hellbraun verfärbtes (±)-2-Azabicyclo[2.2.1]hept-5-en-3-on (Reinheit 98,4 % (HPLC)) erhalten, entsprechend 74 % Ausbeute.

## Patentansprüche

1. Verfahren zur Aufreinigung einer aus einem wäßrigen Herstellprozeß stammenden wäßrigen Reaktionslösung von (±)-2-Azabicyclo[2.2.1]hept-5-en-3-on durch Extrahieren der Reaktionslösung mit einem organischen Lösungsmittel,
**dadurch gekennzeichnet,**
**daß** man nach Phasentrennung die produkthaltige organische Phase mit Wasser vermischt und anschließend phasentrennt, wobei man eine Lösung von (±)-2-Azabicyclo[2.2.1]hept-5-en-3-on in Wasser erhält.

2. Verfahren nach Anspruch 1,
**dadurch gekennzeichnet,**
**daß** man die einzelnen Extraktionsschritte bis zu achtmal hintereinander ausführt.

3. Verfahren nach Anspruch 1,
**dadurch gekennzeichnet,**
**daß** man vor der zweiten Extraktion die organische Phase ggf. unter vermindertem Druck einengt.

4. Verfahren nach Anspruch 1,
**dadurch gekennzeichnet,**
**daß** man vor der ersten Extraktion die wäßrige Phase mit Natriumchlorid versetzt.

5. Verfahren nach Anspruch 1,
**dadurch gekennzeichnet,**
**daß** man aus der finalen wäßrigen Produktphase das (±)-2-Azabicyclo[2.2.1]hept-5-en-3-on durch Eindampfen isoliert.

6. Verfahren nach Anspruch 1,
**dadurch gekennzeichnet,**
**daß** man als organisches Lösungsmittel Methylenchlorid, Methylisopropylketon, Nitromethan, Nitroethan, 1- und 2-Nitropropan verwendet.

7. Verfahren nach Anspruch 1,
**dadurch gekennzeichnet,**
**daß** man als organisches Lösungsmittel MTBE verwendet.

## Claims

1. Process for the purification of an aqueous reaction solution of (±)-2-azabicyclo[2.2.1]hept-5-en-3-one originating from an aqueous preparative process, by extraction of the reaction solution with an organic solvent, **characterized in that**, after phase separation, the product-containing organic phase is mixed with water and the phases are then separated to give an aqueous solution of (±)-2-azabicyclo[2.2.1]-hept-5-en-3-one.

2. Process according to Claim 1, **characterized in that** the individual extraction steps are carried out up to eight times in succession.

3. Process according to Claim 1, **characterized in that** the organic phase is concentrated, optionally under reduced pressure, before the second extraction.

4. Process according to Claim 1, **characterized in that** sodium chloride is added to the aqueous phase before the first extraction.

5. Process according to Claim 1, **characterized in that** the (±)-2-azabicyclo[2.2.1]hept-5-en-3-one is isolated from the final aqueous product phase by evaporation.

6. Process according to Claim 1, **characterized in that** the organic solvent used is methylene chloride, methyl tert-butyl ether, chloroform, methyl isobutyl ketone, methyl isopropyl ketone, nitromethane, nitroethane or 1- or 2-nitropropane.

7. Process according to Claim 1, **characterized in that** MTBE is used as the organic solvent.

## Revendications

1. Procédé de purification d'une solution réactionnelle aqueuse provenant d'un procédé de préparation par voie aqueuse de (±)-2-azabicyclo[2.2.1]hept-5-én-3-one par extraction de la solution réactionnelle avec un solvant organique, **caractérisé en ce qu'**après la séparation des phases, on mélange la phase organique contenant le produit avec de l'eau, puis on sépare les phases, ce qui permet d'obtenir une solution de (±)-2-azabicyclo[2.2.1]hept-5-én-3-one dans de l'eau.

2. Procédé selon la revendication 1, **caractérisé en ce qu'**on réalise les différentes étapes d'extraction consécutivement, jusqu'à huit fois.

3. Procédé selon la revendication 1, **caractérisé en ce qu'**avant la deuxième extraction, on concentre la phase organique, le cas échéant sous pression réduite.

4. Procédé selon la revendication 1, **caractérisé en ce qu'**on mélange avant la première extraction la phase aqueuse avec du chlorure de sodium.

5. Procédé selon la revendication 1, **caractérisé en ce qu'**on isole la (±)-2-azabicyclo[2.2.1]hept-5-én-3-one par concentration par évaporation de la phase de produit aqueuse finale.

6. Procédé selon la revendication 1, **caractérisé en ce qu'**on utilise comme solvant organique le chlorure de méthylène, le méthyl-tert-butyléther, le chloroforme, la méthylisobutylcétone, la méthylisopropylcétone, le nitrométhane, le nitroéthane, le 1-nitropropane et le 2-nitropropane.

7. Procédé selon la revendication 1, **caractérisé en ce qu'**on utilise comme solvant organique le MTBE.
